(19) **Europäisches Patentamt / European Patent Office / Office européen des brevets**

(11) **EP 4 685 242 A1**

(12) **EUROPEAN PATENT APPLICATION**
published in accordance with Art. 153(4) EPC

(43) Date of publication:
**28.01.2026 Bulletin 2026/05**

(21) Application number: **24722664.0**

(22) Date of filing: **21.03.2024**

(51) International Patent Classification (IPC):
***C12Q 1/6883*** (2018.01)    ***G16B 20/20*** (2019.01)

(52) Cooperative Patent Classification (CPC):
**C12Q 1/6883; G16B 20/20;** C12Q 2600/106;
C12Q 2600/156

(86) International application number:
**PCT/ES2024/070184**

(87) International publication number:
**WO 2024/194515 (26.09.2024 Gazette 2024/39)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **23.03.2023 ES 202330245**

(71) Applicants:
• **Baigene, S.L.**
  **01510 Vitoria-Gasteiz Alava (ES)**
• **Santxa Research S.L.U.**
  **01008 Vitoria-Gasteiz (ES)**

(72) Inventors:
• **AZNAR OVIEDO, Jose María**
  **01510 Vitoria-Gasteiz (Araba/Álava) (ES)**
• **CELORRIO HERRERA, David**
  **01510 Vitoria-Gasteiz (Araba/Álava) (ES)**

• **GARCÍA FERNÁNDEZ, Saínza**
  **01008 Vitoria-Gasteiz (Araba/Álava) (ES)**
• **GIMENO LLUCH, Irene**
  **01008 Vitoria-Gasteiz (Araba/Álava) (ES)**
• **JORQUERA CUEVAS, Cristina**
  **01008 Vitoria-Gasteiz (Araba/Álava) (ES)**
• **SÁNCHEZ ARIZMENDIARRIETA, Pello**
  **01008 Vitoria-Gasteiz (Araba/Álava) (ES)**
• **BEITIA SAN VICENTE, Maider**
  **01008 Vitoria-Gasteiz (Araba/Álava) (ES)**
• **DELGADO SAN VICENTE, Diego**
  **01008 Vitoria-Gasteiz (Araba/Álava) (ES)**
• **SÁNCHEZ ÁLVAREZ, Mikel**
  **01008 Vitoria-Gasteiz (Araba/Álava) (ES)**

(74) Representative: **Pons IP**
**Glorieta Rubén Darío 4**
**28010 Madrid (ES)**

Remarks:
The complete document including Reference
Table(s) and the Sequence Listing(s) can be
downloaded from the EPO website

(54) **METHOD FOR OBTAINING USEFUL DATA FOR THE PREDICTION OF A SUBJECT'S RESPONSE TO PLATELET-RICH PLASMA TREATMENT**

(57)    The present invention relates to an *in vitro* method of obtaining useful data for estimating a subject's probability of responding positively to Platelet Rich Plasma (PRP) treatment, comprising the analysis of a series of genetic polymorphisms together with an environmental variable characteristic of the subject. The present invention also relates to a kit comprising the means necessary to detect the genetic polymorphisms and the use of said kit in the aforementioned *in vitro* method.

**EP 4 685 242 A1**

## Description

The present invention relates to an *in vitro* method for obtaining useful data for calculating the probability of a subject responding positively to treatment with Platelet Rich Plasma (PRP), falling within the field of Personalised Medicine. Specifically, this method is based on the analysis of a series of genetic polymorphisms, as well as other environmental data of the subject, which together are useful and allow the prediction of the response to treatment with PRP.

## BACKGROUND ART

PRP is defined as a volume of autologous plasma containing a concentration of platelets above the baseline level. It is a biological and autologous therapy that uses the patient's own plasma and platelet-derived growth factors and other biomolecules for regenerative purposes. These act on tissues by influencing different biological processes. Thus, they help to promote the repair and regeneration of target tissues and structures through modulating effects on inflammation or cell modulation, among others. These properties contribute to promote an appropriate biological environment to promote reparative processes.

In recent years, the use of PRP has become widespread in various medical specialties such as dermatology, aesthetic medicine and dentistry, among others, being musculoskeletal pathologies where the use of PRP is most widely implemented. These pathologies are defined as injuries affecting the locomotor system (muscles, tendons, bones, nerves, ligaments, cartilage and other structures close to the joints). Their origin is usually due to the result of misuse or the action of certain factors on a continuous basis, generally causing chronic pathologies that mainly affect the joints.

Both in Spain and in the European Union, they are the most frequent occupational health problem and represent one of the main causes of work absenteeism. These pathologies have increased exponentially in recent years, affecting a large part of the population. Due to the increase in life expectancy in today's society, musculoskeletal pathologies have become one of the main causes of disability and chronic pain. The economic impact on society is considerable, representing 0.5 to 2 % of GDP.

Current treatments for this type of pathology can be divided into conservative treatments and surgical interventions. The former range from physiotherapeutic treatments to pharmacological therapy, either oral such as anti-inflammatories and analgesics, to cortico-anaesthetic or hyaluronic acid infiltrations depending on the pathology. However, these treatments are focused on relieving the symptoms, but not on repairing the damage caused or slowing down degeneration. When these treatments are not effective, the only solution for these patients is surgery with all the risks and inconveniences that it entails. The main surgical procedures resulting from these pathologies are hip or knee replacement (arthroplasties), which are performed when the level of joint degeneration is irreparable with conservative treatments.

For several years now, regenerative medicine has emerged in the medical field for the treatment of pathologies of the musculoskeletal system. This type of therapy aims to be an alternative for patients who are susceptible to surgery and who have not responded to conventional treatments. In other words, an intermediate step between conservative treatments and surgery, with PRP being the most representative technique in this field, as defined above. The clinical results obtained with this therapy are very positive and promising, with great recovery and patient satisfaction, although there is still a wide margin for improvement in the application of PRP as there are several factors that influence the effectiveness of PRP such as the product or the application protocol.

However, one of the variables that can most influence the clinical outcome of the treatment is the patient's biological characteristics, which can determine both the molecular composition of the PRP and the expression of the cellular receptors that determine the cellular response to the biological products, and a defective expression could limit the efficacy of the treatment. Both the synthesis of PRP molecules and the synthesis and expression of receptors is conditioned by the patient's genetic background. Knowing both the genes involved and the appropriate genetic background would help not only to predict PRP response but also to optimise treatment.

In this sense, it is clear that individual variability is a key factor in obtaining a greater or lesser response to treatment. In this interindividual variability, recent studies have shown how genetic polymorphisms present in genes responsible for the synthesis of these factors may be influencing their expression and concentration. Studies in cancer, cardiovascular therapies and other diseases have reported data directly linking different genetic endowments with variability in response among individuals. Specifically in regenerative medicine, different polymorphic positions have been discovered as possible differentiating factors.

Various methods have been described that are related to the extraction, composition and forms and/or means of PRP use. In the same way, genetic polymorphisms have been associated with different expression or concentration in plasma of the components of PRP. Similarly, it has recently been published the association of different polymorphisms in the PDGFBR gene with the response to PRP treatment in sports pathologies (Szyluk K, et al. 2022, J Clin Med.,11(21):6362. doi: 10.3390/jcm11216362. PMID: 36362590).

However, the methods described in the prior art cannot reliably predict a subject's risk of not responding to PRP treatment. Thus, in light of the above, there is a need for methods that have prognostic utility in predicting a patient's response to PRP.

**DESCRIPTION OF THE INVENTION**

The present invention discloses a method of obtaining useful *in vitro* data for estimating a subject's likelihood of responding positively to PRP treatment.

For the development of said method, the inventors relied on the use of the environmental variable age range together with genetic variables comprising the analysis of one or several genetic polymorphisms of the GH1, SOD2, UCP2, GCKR genes, as well as their combinations with polymorphisms of the IGFR-1, VEGFR2, ADRB2 genes. The implementation of an algorithm that takes into account the previously mentioned environmental and genetic variables makes it possible to obtain a value of the risk of a subject responding positively to treatment with PRP.

The algorithm of the present invention takes into account both genetic and environmental variables to calculate a subject-specific probability value.

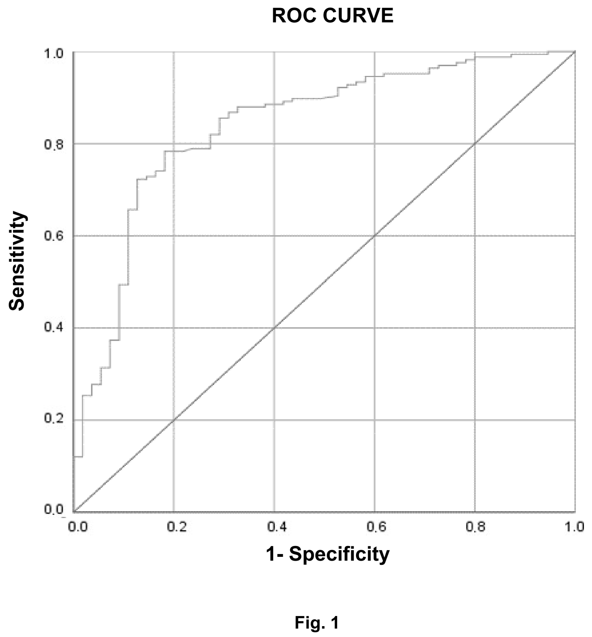

The inventors have observed that the application of this approach in patients has a high sensitivity and specificity, with the method of the invention having good predictive ability, as shown by analysis of ROC curves (for its acronym in English *Receiver Operating Characteristic*) (Figure 1; Tables 7, 8 and 9).

Thus, the present invention provides an innovative approach in the area of personalized medicine with multiple associated advantages: it allows to obtain useful data to know the probability of a patient to respond to PRP treatment with a high sensitivity and specificity; it allows to take decisions prior to treatment such as avoiding such treatment in patients who have shown a low probability of success after the application of the present invention and to opt for other therapeutic options. In addition, it can also be useful when boosting PRP treatment to try to increase the chances of treatment success by emphasising other related variables. Likewise, it allows to know the probability of a patient to respond positively to PRP treatment prior to receiving it.

Method of the invention

In a first aspect, the present invention relates to an *in vitro* method of obtaining useful data for estimating the probability of a subject to respond positively to PRP treatment, hereinafter "the method of the invention", comprising the following steps:

(a) analyse in an isolated biological sample from the subject at least one genetic polymorphism selected from the list consisting of rs2665802 of the GH1 gene, rs4880 of the SOD2 gene, rs660339 of the UCP2 gene, and rs1260326 of the GCKR gene, where each such polymorphism may be replaced by another polymorphism that is in linkage disequilibrium with it;

(b) collect the environmental variable 'age' of the subject,

(c) assign a $\beta$-value to the genetic polymorphisms analysed in step (a) according to Table 2, and assign a $\beta$-value to the subject's environmental variable collected in step (b) according to Table 2; and

(d) calculate a P-value for a subject's probability of responding positively to PRP treatment, by applying the algorithm:

$$P = \frac{1}{1 + e^{-(\beta_o + \beta_1 x)}}$$

wherein,

$\beta_0$ is 0.662
$\beta_1 x$ is the sum of all $\beta$ values assigned in step (c).

As used in the present invention, the term *"in vitro"* refers to the method of the invention being performed outside the body of the subject.

In the context of the present invention, the term "Platelet Rich Plasma" or "PRP" refers to any plasma preparation that has a similar or higher concentration of platelets than the blood sample from which the plasma was derived, as well as serum and other preparations derived from both PRP and whole blood.

These products are used in the treatment of pathologies that require tissue repair due to their biological characteristics which give them great therapeutic potential. The processes triggered by the effector biomolecules contained in these products favour the repair and regeneration of the target tissue, as well as the symptomatic improvement of the patient. The efficacy of PRP and its derivative products depends on factors such as the type of product, the application protocol or the biological characteristics of the patient. Because of this, there are patients who already have a natural predisposition to respond positively or not to treatment with this type of product.

In the present invention, the phrase "estimate a subject's likelihood of responding positively to PRP treatment", refers to estimating or inferring a subject's likelihood of symptomatologic improvement of their pathological condition following PRP treatment. Assessing whether a subject's pathological condition improves symptomatically is routine practice for the person skilled in the art. In the present invention, obtaining data useful for calculating such a probability comprises the application of an algorithm that takes into account both the environmental variable 'age' and the genetic variables, in particular, genetic polymorphisms, of the subject.

In the present invention the algorithm of the method of the invention is considered predictive when the AUCROC (AUC-Area UnderCurve; ROC-Receiver Operating Characteristic) is greater than 0.5. The AUCROC is defined as the probability of correctly classifying a pair of case and control individuals, randomly selected from the population, by the results or values obtained by applying the algorithm to them.

The AUCROC by convention is between 0.5 and 1: the closer the AUCROC value of a variable is to 1, the more accurate and predictive it is considered to be. The sensitivity of the diagnostic test is the probability of obtaining a positive result when the individual responds positively to the intervention, in the present invention, positive response to PRP treatment. The specificity of a test indicates the probability of obtaining a negative result when the individual does not respond positively to the intervention, in the present invention, the application of PRP.

The term "subject" in the present invention refers to any individual susceptible to receive the application of the PRP, or to any individual in whom it is of interest to know the probability of a positive response after undergoing such an intervention. The terms "patient" or "subject" are used interchangeably in the present invention. Examples of "subjects" or "patients" include, but are not limited to, mammals, preferably domestic and farm animals (such as cows, horses, pigs, sheep, goats, dogs, cats, rodents, etc.), non-human primates and humans, more preferably, a human being of either sex or age.

In the present invention, the response to treatment with PRP can occur after administration in any organ or tissue of the subject to treat any pathology that requires action on a target tissue. Preferably, musculoskeletal pathologies. More preferably, in musculoskeletal pathologies involving joint degeneration.

The administration of PRP can be carried out by any of the methods known in the state of the art, such as parenteral, subcutaneous, intra-articular, intra-osseous, intrameniscal, intratendinous, peritendinous, intraligamentous, intramuscular, intraneural or perineural, among others.

Thus, in a preferred embodiment, alone or in combination with the other preferred embodiments, the subject is to undergo PRP treatment. In another more preferred embodiment, the administration of the PRP to the subject is in a joint. Examples of joints, include, but are not limited to, knee, ankle, hip, shoulder and elbow. Thus, in another even more preferred embodiment, the joint is selected from the list consisting of knee, ankle, hip, shoulder and elbow.

### *Step (a) of the method of the invention: analysis of genetic polymorphisms*

A first stage of the method of the invention [step (a)] comprises analysing in a biological sample isolated from the subject, at least one genetic polymorphism selected from the list consisting of rs2665802 of the GH1 gene, rs4880 of the SOD2 gene, rs660339 of the UCP2 gene, and rs1260326 of the GCKR gene, wherein each such polymorphism may be replaced by another polymorphism that is in linkage disequilibrium with it.

As used in the present invention, the term "genetic polymorphism" refers to a variation in the nucleotide sequence of the deoxyribonucleic acid (DNA) chain that has a frequency of at least 1% in individuals of a population. Genetic polymorphisms can be single or multiple nucleotide variations. Single nucleotide polymorphisms, or SNPs, generally give rise to two alleles.

The terms "polynucleotide", "nucleotide sequence", "sequence of nucleotides", "nucleic acid" and "oligonucleotide" are used interchangeably herein and refer to a polymeric form of nucleotides of any length, which may or may not be chemically or biochemically modified.

In the present invention, the term "analyse" referring to "genetic polymorphism(s)" refers to detecting such polymorphism(s), determining their genotype(s).

The genetic polymorphism rs2665802 of the GH1 gene, refers to a SNP located at position 63917670 on homo sapiens chromosome 17 (homo sapiens chromosome 17 sequence GenBank accession number: NC_000017.11:63917669). Genotypes can be homozygous for adenine (A:A), heterozygous adenine:thymine (A:T) or homozygous for thymine (T:T).

The genetic polymorphism rs4880 of the SOD2 gene, refers to a SNP located at position 159692840 on chromosome 6 of homo sapiens (homo sapiens chromosome 6 sequence GenBank accession number:

NC_000006.12:159692839). Genotypes can be homozygous for adenine (A:A), heterozygous adenine:guanine (A:G) or homozygous for guanine (G:G).

The genetic polymorphism rs660339 of the UCP2 gene, refers to a SNP located at position 73978059 on chromosome 11 of homo sapiens (homo sapiens chromosome 11 sequence GenBank accession number: NC_000011.10:73978058). Genotypes can be homozygous for adenine (A:A), heterozygous adenine:guanine (A:G) or homozygous for guanine (G:G).

The genetic polymorphism rs1260326 of the GCKR gene, refers to a SNP located at position 27508073 on chromosome 2 of homo sapiens (homo sapiens chromosome 11 sequence GenBank accession number: NC_000002.12:27508072). Genotypes can be homozygous for cytosine (C:C), heterozygous: cytosine:thymine (C:T) or homozygous for thymine (T:T).

In a preferred embodiment of the method of the invention, alone or in combination with the other previous preferred embodiments of the method of the invention, step (a) comprises analyzing at least two genetic polymorphisms selected from the list consisting of rs2665802 of the GH1 gene, rs2665802 of the GH1 gene, rs4880 of the SOD2 gene, rs660339 of the UCP2 gene, and rs1260326 of the GCKR gene, rs4880 of the SOD2 gene, rs660339 of the UCP2 gene, and rs1260326 of the GCKR gene, wherein each of said polymorphisms can be replaced by another polymorphism that is in linkage disequilibrium with it.

In a more preferred embodiment, alone or in combination with the other preferred embodiments, step (a) comprises testing for the rs2665802 genetic polymorphism of the GH1 gene and the rs4880 genetic polymorphism of the SOD2 gene.

In another more preferred embodiment, alone or in combination with the other preferred embodiments, step (a) comprises testing for the rs2665802 genetic polymorphism of the GH1 gene and the rs660339 genetic polymorphism of the UCP2 gene.

In another more preferred embodiment, alone or in combination with the other preferred embodiments, step (a) comprises testing for the rs2665802 genetic polymorphism of the GH1 gene and the rs1260326 genetic polymorphism of the GCKR gene.

In a more preferred embodiment, alone or in combination with the other preferred embodiments, step (a) comprises analysing the rs4880 genetic polymorphism of the SOD2 gene and the rs660339 genetic polymorphism of the UCP2 gene.

In a more preferred embodiment, alone or in combination with the other preferred embodiments, step (a) comprises testing for the rs4880 genetic polymorphism of the SOD2 gene and the rs1260326 genetic polymorphism of the GCKR gene.

In a more preferred embodiment, alone or in combination with the other preferred embodiments, step (a) comprises analysing the rs660339 genetic polymorphism of the UCP2 gene and the rs1260326 genetic polymorphism of the GCKR gene.

In another preferred embodiment, alone or in combination with the other preferred embodiments, step (a) comprises testing for at least three genetic polymorphisms consisting of the rs2665802 polymorphism of the GH1 gene and two selected from the list consisting of the rs4880 polymorphism of the SOD2 gene, rs660339 of the UCP2 gene, and rs1260326 of the GCKR gene, where each of these polymorphisms can be replaced by another polymorphism that is in linkage disequilibrium with it.

In another preferred embodiment, alone or in combination with the other preferred embodiments, step (a) comprises testing at least three genetic polymorphisms selected from the list consisting of rs2665802 of the GH1 gene, rs4880 of the SOD2 gene, rs660339 of the UCP2 gene, and rs1260326 of the GCKR gene, wherein each such polymorphism can be replaced by another polymorphism that is in linkage disequilibrium with it.

In another more preferred embodiment, alone or in combination with the other preferred embodiments, step (a) comprises testing four genetic polymorphisms selected from the list consisting of rs2665802 of the GH1 gene, rs4880 of the SOD2 gene, rs660339 of the UCP2 gene, and rs1260326 of the GCKR gene, wherein each such polymorphism can be replaced by another polymorphism that is in linkage disequilibrium with it. Even more preferably, the genetic polymorphisms consist of rs2665802 of the GH1 gene, rs4880 of the SOD2 gene, rs660339 of the UCP2 gene, and rs1260326 of the GCKR gene.

In addition to the analysis of at least one, at least two, at least three, at least four, of the genetic polymorphisms selected from the list consisting of the rs2665802 polymorphism of the GH1 gene, rs4880 of the SOD2 gene, rs660339 of the UCP2 gene, and rs1260326 of the GCKR gene, where each of those polymorphisms can be replaced by another polymorphism that is in linkage disequilibrium with it, in particular, in addition to the analysis of the genetic polymorphisms rs2665802 of the GH1 gene, rs4880 of the SOD2 gene, rs660339 of the UCP2 gene, and rs1260326 of the GCKR gene, step (a) of the method of the invention can comprise analysing at least one genetic polymorphism selected from the list consisting of the rs2229765 polymorphism of the IGFR-1 gene, rs1870377 of the VEGFR2 gene, and rs1042714 of the ADRB2 gene, wherein each such polymorphism may be replaced by another polymorphism that is in linkage disequilibrium with it.

The genetic polymorphism rs2229765 of the IGFR-1 gene, refers to a SNP located at position 98934996 on chromosome 15 of homo sapiens (homo sapiens chromosome 15 sequence GenBank accession number: NC_000015.10:98934995). Genotypes can be homozygous for adenine (A:A), heterozygous adenine:guanine (A:G) or homozygous for guanine (G:G).

The genetic polymorphism rs1870377 of the VEGFR2 gene, refers to a SNP located at position 55106807 on chromosome 4 of homo sapiens (homo sapiens chromosome 4 sequence GenBank accession number: NC_000004.12:55106806). Genotypes can be homozygous for adenine (A:A), heterozygous adenine:thymine (A:T) or homozygous for thymine (T:T).

The genetic polymorphism rs1042714 of the ADRB2 gene, refers to a SNP located at position 148826910 on chromosome 5 of homo sapiens (homo sapiens chromosome 5 sequence GenBank accession number: NC_000005.10:148826909). Genotypes can be homozygous for cytosine (C:C), heterozygous cytosine:guanine (C:G) or homozygous for guanine (G:G).

Thus, in a preferred embodiment of the method of the invention, alone or in combination with the other preferred embodiments, step (a) further comprises analysing at least one genetic polymorphism selected from the list consisting of the rs2229765 polymorphism of the IGFR-1 gene, the rs1870377 polymorphism of the VEGFR2 gene, and the rs1042714 polymorphism of the ADRB2 gene, rs1870377 of the VEGFR2 gene, and rs1042714 of the ADRB2 gene, wherein each such polymorphism may be replaced by another polymorphism that is in linkage disequilibrium with it.

In another preferred embodiment of the method of the invention, alone or in combination with the other preferred embodiments, step (a) further comprises testing for at least two, at least three, genetic polymorphisms selected from the list consisting of the rs2229765 polymorphism of the IGFR-1 gene, rs1870377 of the VEGFR2 gene, and rs1042714 of the ADRB2 gene, wherein each such polymorphism may be replaced by another polymorphism that is in linkage disequilibrium with it.

In another preferred embodiment of the method of the invention, alone or in combination with the other preferred embodiments, step (a) comprises analyzing at least seven genetic polymorphisms selected from the list consisting of rs2229765 of the IGFR-1 gene, rs1870377 of the VEGFR2 gene, rs1042714 of the ADRB2 gene, rs2665802 of the GH1 gene, rs4880 of the SOD2 gene, and rs660339 of the UCP2 gene, rs1042714 of the ADRB2 gene, rs2665802 of the GH1 gene, rs4880 of the SOD2 gene, rs660339 of the UCP2 gene, and rs1260326 of the GCKR gene, wherein each such polymorphism may be replaced by another polymorphism that is in linkage disequilibrium with it, including any combination thereof.

In another more preferred embodiment of the method of the invention, alone or in combination with the other preferred embodiments, step (a) comprises analysing the genetic polymorphisms rs2229765 of the IGFR-1 gene, rs1870377 of the VEGFR2 gene, rs1042714 of the ADRB2 gene, rs2665802 of the GH1 gene, rs4880 of the SOD2 gene, rs660339 of the UCP2 gene, rs1260326 of the GCKR gene.

The phrase "polymorphisms of the invention" is used herein to refer to at least one genetic polymorphism selected from among rs2665802 of the GH1 gene, rs4880 of the SOD2 gene, rs660339 of the UCP2 gene, and rs1260326 of the GCKR gene, wherein each such polymorphism may be replaced by another polymorphism that is in linkage disequilibrium with it, and/or to any combination of one or more of the above genetic polymorphisms with at least one genetic polymorphism selected from rs2229765 of the IGFR-1 gene, rs1870377 of the VEGFR2 gene, and rs1042714 of the ADRB2 gene, where each such polymorphism can be replaced by another polymorphism that is in linkage disequilibrium with it.

In the present invention, the genetic polymorphisms are SNPs, so the terms "polymorphisms of the invention and "SNPs of the invention" may be used interchangeably throughout the document.

Table 1 shows some of the genetic polymorphisms that can be included as genotypic variables within the method of the invention.

| Gene | Polymorphism | Genotypes | Location |
|---|---|---|---|
| IGFR-1 | rs2229765 | A:A | Chromosome 15:98934996 |
| | | A:G | NC_000015.10:g.98934996G>A |
| | | G:G | |
| VEGFR2 | rs1870377 | A:A | Chromosome 4:55106807 |
| | | A:T | NC_000004.12:g.55106807T>A |
| | | T:T | |

(continued)

| Gene | Polymorphism | Genotypes | Location |
|------|-------------|-----------|----------|
| ADRB2 | rs1042714 | C:C | Chromosome 5:148826910 |
| | | C:G | NC_000005.10:g.148826910G>C |
| | | G:G | |
| GH1 | rs2665802 | A:A | Chromosome 17:63917670 |
| | | A:T | NC_000017.11:g.63917670A>T |
| | | T:T | |
| SOD2 | rs4880 | A:A | Chromosome 6:159692840 |
| | | A:G | NC_000006.12:g.159692840A>G |
| | | G:G | |
| UCP2 | rs660339 | A:A | Chromosome 11:73978059 |
| | | A:G | NC_000011.10:g.73978059G>A |
| | | G:G | |
| GCKR | rs1260326 | C:C | Chromosome 2:27508073 |
| | | C:T | NC_000002.12:g.27508073T>C |
| | | T:T | |

As indicated in the present invention, each such polymorphism can be replaced by another polymorphism that is in linkage disequilibrium with it.

The term "linkage disequilibrium" refers to the property of some genes or DNA markers not to segregate independently, i.e. they have a recombination frequency of less than 50%. This is often due to the fact that the two loci involved are on the same chromosome, which makes it impossible to transfer them to the progeny in a random manner with the separation of the chromosomes in anaphase. In the present invention, this refers to those genetic polymorphisms which, due to their physical proximity within a chromosome, occur together more frequently than would be expected by chance. A genetic polymorphism that is in linkage disequilibrium with another has the same ability or gives equivalent information, in the present invention, regarding the prediction of a subject's likelihood of responding positively to PRP treatment, since by the very definition, both are inherited together.

A measure of linkage disequilibrium between two genetic markers is defined as "r2". Two genetic polymorphisms that have not been separated by recombination (total linkage disequilibrium), show an r2=1. In the present invention, the polymorphisms that are in linkage disequilibrium with the genetic polymorphisms rs2229765 of the IGFR-1 gene, rs1870377 of the VEGFR2 gene, rs1042714 of the ADRB2 gene, rs2665802 of the GH1 gene, rs4880 of the SODR gene, rs4880 of the SODR gene, rs4880 of the SODR gene, rs4880 of the SODR gene and rs4880 of the SODR gene, rs4880 of the SOD2 gene, rs660339 of the UCP2 gene, rs1260326 of the GCKR gene, preferably have a linkage disequilibrium measure r2 $\geq$ 0.8, more preferably a linkage disequilibrium measure r2 $\geq$ 0.9. Calculation of linkage disequilibrium is routine practice for the person skilled in the art.

The analysis of the polymorphisms of the invention can be performed by any method known to the person skilled in the art for that purpose. For example, it can be performed by genotyping kits, sequencing or by PCR (polymerase chain reaction) amplification and subsequent restriction enzyme analysis or by real-time PCR. Genotyping kits may contain fluorophore-labelled oligonucleotides and may require hybridisation of these with a biological sample isolated from a subject.

Thus, in the method of the invention, the analysis of the polymorphisms of the invention of step (a) is carried out by any method known to the person skilled in the art for that purpose. In a preferred embodiment, alone or in combination with the other preferred embodiments, it is carried out by amplification, more preferably PCR amplification, and/or sequencing.

In another preferred embodiment of the method of the invention, the analysis of the polymorphisms of the invention of step (a) is carried out using a genotyping kit.

In another preferred embodiment of the method of the invention, alone or in combination with the other preferred embodiments, the analysis of the polymorphisms of the invention of step (a) comprises the use of probes and/or primers that specifically detect and/or amplify such polymorphisms.

In a more preferred embodiment, the primers and/or probes comprise, or consist of, the nucleotide sequences: SEQ ID NO: 1, SEQ ID NO: 2, SEQ ID NO: 3, SEQ ID NO: 4, SEQ ID NO: 5, SEQ ID NO: 6, , SEQ ID NO: 7, SEQ ID NO: 8, SEQ ID NO: 9, SEQ ID NO: 10, SEQ ID NO: 11, , SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, and/or any combination thereof.

SEQ ID NO: 1
ACAGTGAACGAGGCCGCA
SEQ ID NO: 2
GACAATTGAACTCCTTCATCACAGA

Sequences SEQ ID NO:1 and SEQ ID NO: 2, correspond to the sequences of the specific primers to amplify the rs2229765 polymorphism of the IGFR-1 gene.

SEQ ID NO: 3
AGCTTCGTTGAGAAACTCAATCCTT
SEQ ID NO: 4
GCTTCGTTGAGAAACTCAATCCTC

Sequences SEQ ID NO:3 and SEQ ID NO: 4 correspond to the sequences of the specific probes to specifically detect the rs2229765 polymorphism of the IGFR-1 gene.

SEQ ID NO: 5
TGCGCTGTTATCTCTTTCTTTCTTATATAGCCA
SEQ ID NO: 6
CTCCACACACTTCTTCTCCATTCTTCAC

Sequences SEQ ID NO:5 and SEQ ID NO: 6, correspond to the sequences of the specific primers to amplify the rs1870377 polymorphism of the VEGFR2 gene.

SEQ ID NO: 7
GGGTTTGTCACTGAGACAGCA
SEQ ID NO: 8
GGGTTTGTCACTGAGACAGCT

Sequences SEQ ID NO:7 and SEQ ID NO: 8 correspond to the sequences of the specific probes to specifically detect the rs1870377 polymorphism of the VEGFR2 gene.

SEQ ID NO: 9
GAAGCCATGCGCCGGAC
SEQ ID NO: 10
GACGATGCCCATGCCC

Sequences SEQ ID NO:9 and SEQ ID NO: 10, correspond to the sequences of the specific primers to amplify the rs1042714 polymorphism of the ADRB2 gene.

SEQ ID NO: 11
CCCACACACCTCGTCCCTTTG
SEQ ID NO: 12
CCCACACACCTCGTCCCTTTC

Sequences SEQ ID NO:11 and SEQ ID NO: 12 correspond to the sequences of the specific probes to specifically detect the rs1042714 polymorphism of the ADRB2 gene.

SEQ ID NO: 13
TGAGTTCTCTTGGGTCAGGGC
SEQ ID NO: 14
CCCACTGACTTTGAGAGAGCTG

Sequences SEQ ID NO:13 and SEQ ID NO: 14, correspond to the sequences of the specific primers to amplify the rs2665802 polymorphism of the GH1 gene.

SEQ ID NO: 15
TGCTGCCCTCTCTTTTTAGCAGT
SEQ ID NO: 16
TGCTGCCCTCTTTTTAGCAGA

Sequences SEQ ID NO:15 and SEQ ID NO: 16 correspond to the sequences of the specific probes to specifically detect the rs2665802 polymorphism of the GH1 gene.

SEQ ID NO: 17
CTGTGCTTTCTCGTCTTCAGCA
SEQ ID NO: 18
GGCTGTGCTTCTTCTGCCTG

Sequences SEQ ID NO:17 and SEQ ID NO: 18, correspond to the sequences of the specific primers to amplify the rs4880 polymorphism of the SOD2 gene.

SEQ ID NO: 19
GGAGCCCAGATACCCCAAAG
SEQ ID NO: 20
GGAGCCCAGATACCCCAAAA

Sequences SEQ **ID** NO:19 and SEQ ID NO: 20 correspond to the sequences of the specific probes to specifically detect the rs4880 polymorphism of the SOD2 gene.

SEQ ID NO: 21
GGTCAGAATGGTGCCCATCAC
SEQ ID NO: 22
CAGATCCAAGGAGAAAGTCAGG

Sequences SEQ **ID** NO:21 and SEQ ID NO: 22, correspond to the sequences of the specific primers to amplify the rs660339 polymorphism of the UCP2 gene.

SEQ ID NO: 23
CAGTGCGCGCGCTACAGC
SEQ ID NO: 24
CCAGTGCGCGCGCTACAGT

Sequences SEQ **ID** NO:23 and SEQ ID NO: 24 correspond to the sequences of the specific probes to specifically detect the rs660339 polymorphism of the UCP2 gene.

SEQ ID NO: 25
GGGTCCCTTTGTCACGGCT
SEQ ID NO: 26
ACTTATTCTCTGTTAGAAATATATCTAGAAAACTACTACGACCT

Sequences SEQ **ID** NO:25 and SEQ ID NO: 26, correspond to the sequences of the specific primers to amplify the rs1260326 polymorphism of the GCKR gene.

SEQ ID NO: 27
TCTCCTAACAAACTGTTTCACATCTTTTTT
SEQ ID NO: 28
TCTCCTAACAAACTGTTTCACATCTTTTTC

Sequences SEQ ID NO:27 and SEQ ID NO: 28 correspond to the sequences of the specific probes to specifically detect the rs1260326 polymorphism of the GCKR gene.
In a more preferred embodiment of the method of the invention, the analysis of the polymorphisms carried out in step (a) comprises use of probes detecting the polymorphisms of the invention. Preferably, the probes detecting said polymorphisms are selected from the list consisting of: SEQ ID NO: 3, SEQ ID NO: 4, SEQ ID NO: 7, SEQ ID NO: 8, SEQ ID NO: 11, SEQ ID NO: 12, SEQ ID NO: 15, SEQ ID NO: 16, SEQ ID NO: 19, SEQ ID NO: 20, SEQ ID NO: 23, SEQ ID NO: 24, SEQ ID NO: 27, SEQ ID NO: 28, and any combination thereof.

In another preferred embodiment of the method of the invention, alone or in combination with the other preferred embodiments, the analysis of the polymorphisms of the invention of step (a) comprises the use of fluorophore-labelled probes. Examples of fluorophores include, but are not limited to, Alexa fluor, GFP (Green Fluorescent Protein), cerulean, mCherry, FITC, ICG, Cy-5, rhodamine 800, 5-TAMRA, and methylene blue.

The term "sequencing" as used in the present description refers to the determination of the nucleotides of a template nucleic acid and their order.

The term "amplification", as used in the present description, refers to increasing the copy number of a template nucleic acid, where amplification can be performed using fluorescent probes. In a preferred embodiment of the method of the invention, alone or in combination with the other preferred embodiments, amplification takes place by real- time PCR.

The term "template nucleic acid' or 'template' as used herein shall mean description, refers to a single-stranded or double-stranded nucleic acid molecule to be amplified or sequenced.

Increasing the copy number of a template nucleic acid is accomplished by synthesis of complementary DNA under conditions that allow it. Conditions allowing complementary DNA synthesis refers to conditions under which incorporation of nucleotides into a nascent DNA by base complementarity with the template nucleic acid can take place.

The conditions under which sequencing or amplification is performed generally include (a) contacting a template nucleic acid with a polymerase in a mixture further comprising a primer, a bivalent cation, (e.g., Mg2+), and nucleotides, generally dNTPs, and at least one ddNTP, and (b) subjecting the mixture to a temperature sufficient for the polymerase to initiate the incorporation of the nucleotides into the primer by base complementation with the template nucleic acid, resulting in a population of complementary DNA molecules of different sizes. Separation of this population of complementary DNA molecules, usually by electrophoresis, allows the nucleotide sequence to be determined.

The term "primer", as used herein, refers to an oligonucleotide capable of acting as a starting point for DNA synthesis when hybridized to the template nucleic acid. Preferably, the primer is a deoxyribose oligonucleotide. Primers can be prepared by any suitable method, including, for example, but not limited to, cloning and restriction of appropriate sequences and direct chemical synthesis. Primers can be designed to hybridize with specific nucleotide sequences in the template nucleic acid (specific primers) or can be synthesized at random (arbitrary primers).

The term 'specific primer', as used in the present description, refers to a primer whose sequence is complementary to a specific sequence of nucleotides in the template nucleic acid to be amplified or sequenced.

The term 'arbitrary primer' refers to a primer whose sequence is synthesized at random and which is used to initiate DNA synthesis at random positions of the template nucleic acid to be amplified or sequenced. A population of different arbitrary primers is often used. The term "arbitrary primers" refers to a set of primers whose sequence is randomly synthesized and used to initiate DNA synthesis at random positions of the template nucleic acid to be amplified or sequenced.

The term "hybridization" as used in the present description refers to the pairing of two complementary single-stranded DNA molecules to give a double-stranded molecule. Preferably, the complementarity is 100%. That is, in the region of complementarity each nucleotide in one of the two nucleic acid molecules can form hydrogen bonds with a nucleotide present in the other nucleic acid molecule. However, those with normal experience in the field will recognize that two nucleic acid molecules possessing a region of less than 100% complementarity can also hybridize.

The term "nucleotide" as used in the present description refers to an organic molecule formed by the covalent bonding of a pentose, a nitrogenous base and a phosphate group. The term nucleotide includes deoxyribonucleotide triphosphates such as, but not limited to, dATP, dCTP, dITP, dUTP, dGTP, dTTP, or derivatives thereof. The term nucleotide also includes dideoxyribonucleoside triphosphates (ddNTPs), such as, but not limited to, ddATP, ddCTP, ddGTP, 15 ddITP, ddTTP, or derivatives thereof.

According to the present invention, a "nucleotide" or a "primer" can be tagged or labelled by techniques well known in the prior art. Detectable labels include, but are not limited to, radioactive isotopes, fluorescent labels, chemiluminescent labels, bioluminescent labels and enzymatic labels.

The term "biological sample" in the present invention refers to any sample from which DNA can be obtained from the individual from whom such a sample has been obtained, and includes, but is not limited to, biological tissues and/or fluids from an individual obtained by any method known to a person skilled in the art that is suitable for that purpose.

Thus, in a preferred embodiment of the method of the invention, alone or in combination with the other preferred embodiments, the biological sample is derived from any tissue susceptible to DNA extraction. The biological sample could be, for example, but not limited to, a tissue or fluid sample, such as blood, plasma, serum, oral mucosa, bronchoalveolar lavage, lymph or ascitic fluid. In a more preferred embodiment, the biological sample is selected from the list consisting of tissue, oral mucosa, blood, plasma, serum and lymph. Also, the biological sample can be, for example, but not limited to, fresh, frozen, fixed or fixed and embedded in paraffin.

**EP 4 685 242 A1**

*Step (b) of the method of the invention: Collecting data from the subject*

As explained previously in the present description, the algorithm used in the method of the invention that allows the calculation of a subject's probability of responding positively to treatment with PRP is based on the use of genomic variables together with environmental variables of each subject. For this purpose, it is therefore necessary to collect data relating to the said environmental variables of the subject.

Thus, a second stage of the method of the invention [step (b)] comprises collecting the environmental variable 'age' of the subject.

In the present invention, "subject environmental variable" refers to clinical or physiological data that a subject presents in addition to their genetic data. Examples of subject environmental variables include, but are not limited to, age, sex, weight, height, pathology, tissue where surgery is to be performed, blood pressure, blood glucose, etc. Thus, the phrase "collecting a variable from a subject" refers to the collection and/or recording of data relating to the clinical or physiological state of a subject. In the present invention, the environmental variable to be collected is the age of the subject.

As one expert in the field understands, such data collection can be carried out using any methodology or protocol followed by healthcare professionals.

*Step (c) of the method of the invention: assigning a $\beta$-value to the polymorphisms and data of the subject.*

As already mentioned, the method of the invention takes into account genetic variables and the environmental variable 'age' of the subject. Prior to the application algorithm of the invention, a value, in the present invention value $\beta$, is assigned to the polymorphisms analyzed in step (a), and to the environmental variable collected from the subject in step (b) of the method of the invention.

Thus, a third stage of the method of the invention, step (c), comprises assigning a $\beta$ value to the genetic polymorphisms analyzed in step (a) according to Table 2, and assigning a $\beta$ value to environmental variable collected from the subject in step (b) according to Table 2.

**Table 2.** Specific $\beta$ values for each possibility of the variables analyzed. In the case of the genetic polymorphisms analyzed in step (a) of the method, a $\beta$ value is assigned for each polymorphism (according to the subject's genotype for that polymorphism). In the case of the environmental variables collected from the subject in step (b) of the method, a $\beta$-value is assigned for each data collected.

| Variable | Possibility | Values of $\beta$ |
|---|---|---|
| Age ranges | <35 years | 0.159 |
| | 36-49 years | 0.525 |
| | 50-69 years | 1.441 |
| | >69 | 0.000 |
| rs4880 (or any polymorphism in linkage disequilibrium with it) | A:A | 0.107 |
| | G:A | 1.469 |
| | G:G | 0.000 |
| rs660339 (or any polymorphism in linkage disequilibrium with it) | C:C | 1.415 |
| | C:T | -0.241 |
| | T:T | 0.000 |
| rs1260326 (or any polymorphism in linkage disequilibrium with it) | C:C | 0.786 |
| | C:T | -0.382 |
| | T:T | 0.000 |
| rs2229765 (or any polymorphism in linkage disequilibrium with it) | A:A | -1.423 |
| | A:G | -0.669 |
| | G:G | 0.000 |
| rs1042714 (or any polymorphism in linkage disequilibrium with it) | C:C | -1.466 |
| | C:G | -1.992 |
| | G:G | 0.000 |

(continued)

| Variable | Possibility | Values of $\beta$ |
|---|---|---|
| rs1870377 (or any polymorphism in linkage disequilibrium with it) | A:A | -2.380 |
| | A:T | -0.110 |
| | T:T | 0.000 |
| rs2665802 (or any polymorphism in linkage disequilibrium with it) | A:A | 1.173 |
| | A:T | 1.606 |
| | T:T | 0.000 |

Thus, in the case of the genetic polymorphisms analyzed in step (a) of the method of the invention, for each polymorphism, according to the genotype of the subject, a $\beta$ value is assigned. In the case of the environmental variable collected from the subject in step (b). of the method of the invention, a $\beta$ value is assigned according to the age of the subject analyzed.

The sum of the $\beta$ values assigned in this step (c) of the method of the invention, constitutes the $B_1x$ value that forms part of the algorithm applied in the next stage of the method of the invention.

*Step (d) of the method of the invention: Calculation of the P-value by applying an algorithm.*

The next stage of the method of the invention, step (d), comprises calculating the probability P of a subject to respond positively to PRP treatment, by applying the algorithm:

$$P = \frac{1}{1 + e^{-(\beta_o + \beta_1 x)}}$$

wherein,

$\beta_0$ is 0.662
$\beta_1x$ is the sum of all $\beta$ values assigned in step (c).

This algorithm, hereinafter the algorithm of the invention, relates the probability of a subject to respond positively to PRP treatment, according to the genetic polymorphisms previously analyzed and the environmental variable 'age' data previously collected from the subject, through $\beta_1x$.

$\beta_1x$, is the sum of all the $\beta$ values assigned in step (c) to each of the variables: each polymorphism analyzed in step (a) of the method of the invention, according to the genotype, is assigned a $\beta$ value according to Table 2; each data collected from the subject in step (b) of the method of the invention, is also assigned a $\beta$ value according to Table 2. The sum of all the $\beta$ values, constitutes the $B_1x$ value comprised in the algorithm of the invention.

The application of the algorithm of the invention makes it possible to calculate the P-value of a subject's probability of responding positively to PRP treatment. In the present invention, "the P-probability of a subject to respond positively to PRP treatment" (also referred to as "P-value", terms used interchangeably in the present invention), refers to the probability of a subject to respond positively to PRP treatment. The calculated value is in per cent, and can also be interpreted in terms of percentage, by multiplying the calculated value by 100. Thus, the method of the invention allows predicting the probability of a subject to respond positively to PRP treatment. In the present invention it is understood that a subject "responds positively to a treatment" when the subject improves symptomatologically from his pathological condition, i.e. one or more of the symptoms associated with a pathology disappear, diminish or are alleviated.

Kit of the invention and its uses

The implementation of the method of the invention is based, in addition to the collection of environmental variables relating to the subject, on the analysis of genetic polymorphisms, the polymorphisms of the invention. The means used for the analysis of such genetic polymorphisms may be part of a kit.

Thus, in another aspect, the invention relates to a kit, hereinafter the "kit of the invention", comprising the means necessary to analyse *in vitro* in a biological sample isolated from a subject at least one, two three, four or five genetic polymorphisms selected from the list consisting of rs2665802 of the GH1 gene, rs4880 of the SOD2 gene, rs660339 of the UCP2 gene, and rs1260326 of the GCKR gene, wherein each of said polymorphisms can be replaced by another polymorphism that is in linkage disequilibrium with it.

The genetic polymorphisms described in the kit of the invention have been described in previous inventive aspects together with an explanation of the concept "polymorphism that is in linkage disequilibrium with said genetic polymorphisms". All these definitions and explanations are applicable to the kit of the invention.

On the other hand, in a preferred embodiment, the kit of the invention further comprises, in addition, the means necessary to analyze *in vitro,* in the biological sample isolated from the subject, at least one, two, three, or four genetic polymorphisms selected from the list consisting of rs2229765 of the IGFR-1 gene, rs1870377 of the VEGFR2 gene, and rs1042714 of the ADRB2 gene, wherein each of said polymorphisms may be replaced by another polymorphism that is in linkage disequilibrium with it.

In another more preferred embodiment, alone or in combination with the other preferred embodiments, the kit of the invention comprises the means necessary to analyze *in vitro,* in a biological sample isolated from the subject, the genetic polymorphisms rs2665802 of the GH1 gene, rs4880 of the SOD2 gene, rs660339 of the UCP2 gene, rs1260326 of the GCKR gene, rs2229765 of the IGFR-1 gene, rs1870377 of the VEGFR2 gene, and rs1042714 of the ADRB2 gene.

In the present invention, "means necessary to analyze genetic polymorphisms *in vitro"* means all those reagents used to analyze the polymorphisms of the invention *in vitro* (primers, probes, buffers, enzymes, coenzymes, substrates). In addition, the kits can include all the media and containers necessary for their set-up and optimization (plastic tubes, plates, reagents, etc.). The kits can also contain other molecules, genes, proteins or probes of interest to serve as positive and negative controls.

As a person skilled in the field knows, genetic polymorphisms can be analyzed by techniques and methods known in the prior art, including techniques and methods different from those presented here. These methods and techniques have been explained in the previous inventive aspect, and both they and their preferred embodiments are applicable to the kit of the invention.

In a preferred embodiment of the kit of the invention, alone or in combination with the above preferred embodiments, the means necessary to analyze *in vitro* the polymorphisms of the invention comprise the reagents necessary to carry out the amplification technique, preferably PCR, and/or sequencing.

In another preferred embodiment of the kit of the invention, alone or in combination with the above preferred embodiments, the means for analyzing *in vitro* the polymorphisms of the invention comprise primers and/or probes that specifically detect such genetic polymorphisms.

In a more preferred embodiment of the kit of the invention, alone or in combination with the challenge of preferred embodiments, the primers and/or probes comprise, or consist of, the nucleotide sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO:15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, and/or any combination thereof.

More preferably, the means comprise probes that specifically detect the polymorphisms of the invention. Even more preferably, the probes detecting said polymorphisms are selected from the list consisting of the sequences: SEQ ID NO: 3, SEQ ID NO: 4, SEQ ID NO: 7, SEQ ID NO: 8, SEQ ID NO: 11, SEQ ID NO: 12, SEQ ID NO: 15, SEQ ID NO: 16, SEQ ID NO: 19, SEQ ID NO: 20, SEQ ID NO: 23, SEQ ID NO: 24, SEQ ID NO: 27, SEQ ID NO: 28, and any combination thereof.

In a preferred embodiment of the kit of the invention, alone or in combination with the other preferred embodiments, the biological sample is derived from any tissue susceptible to DNA extraction. The biological sample could be, for example, but not limited to, a tissue or fluid sample, such as blood, plasma, serum, oral mucosa, bronchoalveolar lavage, lymph or ascitic fluid. In a more preferred embodiment of the kit of the invention, the biological sample is selected from the list consisting of tissue, oral mucosa, blood, plasma, serum and lymph.

Preferably, the kits further comprise instructions for carrying out the analysis of the polymorphisms of the invention and/or the method of the invention. These instructions may be present in the said kits in a variety of forms, one or more of which may be present in the kit. One form in which these instructions may be present is as printed information on a suitable medium or substrate, e.g., a sheet or sheets of paper on which the information is printed, on the kit packaging, on a package insert, etc. Another medium would be a computer readable medium, e.g. CD, USB, etc., on which the information has been recorded. Another medium that may be present is a website address that can be used via the Internet to access the information at a remote site. Any convenient medium may be present in the kits.

The kit of the invention, comprising the means for analyzing *in vitro* the polymorphisms of the invention, has utility for implementing the method of the invention. Thus, in another aspect, the invention relates to the use of the kit of the invention in the method of the invention.

The terms used to define the kit and use of the kit of the invention have been explained for the method of the invention, and both they and their preferred embodiments are applicable also for the kit of the invention and its use in the method of the invention.

## DESCRIPTION OF THE DRAWINGS

**Figure 1.** ROC curve graph of the prediction model taking into account as variables the genetic polymorphisms rs2665802 of the GH1 gene, rs4880 of the SOD2 gene, rs660339 of the UCP2 gene, rs1260326 of the GCKR gene, rs2229765 of the IGFR-1 gene, rs1870377 of the VEGFR2 gene, rs1042714 of the ADRB2 gene and the environmental variable age range, in which each point of the graph shows a possible cut-off point for the model in which it informs about its specific sensitivity in the ADRB2 gene, rs1042714 of the ADRB2 gene and the environmental variable age range, in which each point of the graph shows a possible cut-off point for the model in which it reports its specific sensitivity at that point (Y-axis) with respect to its 1-specificity (X-axis). Diagonally across the graph (from 0.0 to 1.1) is the diagonal reference line or non-discrimination line. The segments of the diagonal are generated by ties.

**Figure 2.** ROC curve plot of the prediction model taking into account as genetic variable the rs2665802 polymorphism of the GH1 gene and as environmental variable the age range. The diagonal segments are generated by means of ties.

**Figure 3.** ROC curve graph of the prediction model taking into account the rs4880 polymorphism of the SOD2 gene as a genetic variable and age range as an environmental variable. The diagonal segments are generated by means of ties.

**Figure 4.** ROC curve graph of the prediction model taking into account the rs660339 polymorphism of the UCP2 gene as a genetic variable and the age range as an environmental variable. The diagonal segments are generated by means of ties.

**Figure 5.** ROC curve graph of the prediction model taking into account the rs1260326 polymorphism of the GCKR gene as a genetic variable and age range as an environmental variable. The diagonal segments are generated by means of ties.

**Figure 6.** ROC curve graph of the prediction model taking into account as genetic variable the genetic polymorphisms rs2665802 of the GH1 gene and rs4880 of the SOD2 gene, and as environmental variable the age range. The diagonal segments are generated by means of ties.

**Figure 7.** ROC curve graph of the prediction model taking into account as genetic variable the genetic polymorphisms rs2665802 of the GH1 gene and rs660339 of the UCP2 gene, and as environmental variable the age range. The diagonal segments are generated by means of ties.

**Figure 8.** ROC curve graph of the prediction model taking into account as genetic variable the genetic polymorphisms rs2665802 of the GH1 gene and rs1260326 of the GCKR gene, and as environmental variable the age range. The diagonal segments are generated by means of ties.

**Figure 9.** ROC curve graph of the prediction model taking into account as genetic variable the genetic polymorphisms rs4880 of the SOD2 gene and rs660339 of the UCP2 gene, and as environmental variable the age range. The diagonal segments are generated by means of ties.

**Figure 10.** ROC curve graph of the prediction model taking into account as genetic variable the genetic polymorphisms rs4880 of the SOD2 gene and rs1260326 of the GCKR gene, and as environmental variable the age range. The diagonal segments are generated by means of ties.

**Figure 11.** ROC curve graph of the prediction model taking into account as a genetic variable the genetic polymorphisms rs660339 of the UCP2 gene and rs1260326 of the GCKR gene, and as an environmental variable the age range. The diagonal segments are generated by means of ties.

**Figure 12.** ROC curve graph of the prediction model taking into account as genetic variable the genetic polymorphisms rs2665802 of the GH1 gene, rs4880 of the SOD2 gene and rs660339 of the UCP2 gene, and as environmental variable the age range. The diagonal segments are generated by means of ties.

**Figure 13.** ROC curve graph of the prediction model taking into account as genetic variable the genetic polymorphisms rs2665802 of the GH1 gene, rs4880 of the SOD2 gene and rs1260326 of the GCKR gene, and as environmental variable the age range. The diagonal segments are generated by means of ties.

**Figure 14.** ROC curve graph of the prediction model taking into account as a genetic variable the genetic polymorphisms rs2665802 of the GH1 gene, rs660339 of the UCP2 gene and rs1260326 of the GCKR gene, and as an environmental variable the age range. The diagonal segments are generated by means of ties.

**Figure 15.** ROC curve graph of the prediction model taking into account as genetic variable the genetic polymorphisms rs4880 of the SOD2 gene, rs660339 of the UCP2 gene and rs1260326 of the GCKR gene, and as environmental variable the age range. The diagonal segments are generated by means of ties.

**Figure 16.** ROC curve graph of the prediction model taking into account as genetic variable the genetic polymorphisms rs2665802 of the GH1 gene, rs4880 of the SOD2 gene, rs660339 of the UCP2 gene and rs1260326 of the GCKR gene, and as environmental variable the age range. The diagonal segments are generated by means of ties.

**Examples**

In the following, the invention will be illustrated by tests carried out by the inventors, which demonstrate the effectiveness of the invention, including an example of the application of the method of the invention in predicting a subject's probability of responding positively to PRP treatment.

**1. Methodology**

A total of 221 patients who had been treated with PRP by the trauma team of the Arthroscopic Surgery Unit of Dr. Mikel Sanchez were selected. All of them had a buccal swab sample taken using 4N6FLOQSwab-Life Technologies swabs. DNA from the samples was extracted using QIAmp DNA Mini kit (Qiagen), and fluorimetrically quantified using Qubit (Life Technologies). The resulting DNA samples were analyzed by SNP genotyping analysis (GH1 gene rs2665802, SOD2 gene rs4880, UCP2 gene rs660339, GCKR gene rs1260326, IGFR-1 gene rs2229765, VEGFR2 gene rs1870377, ADRB2 gene rs1042714) using the Biomark HD system (Fluidigm). Patients were categorized according to the following environmental factor:

- Age range:

  ○ <35 years
  ○ 36-49 years
  ○ 50-69 years

From the data relating to the environmental variable 'age' and genetic polymorphisms, the inventors applied the following algorithm to obtain a subject's probability of responding positively to PRP treatment (referred to as P or P-value):

$$P = \frac{1}{1 + e^{-(\beta_0 + \beta_1 x)}}$$

wherein,

$\beta_0$ = 0.662
$\beta_1 x$ is the sum of all the $\beta$ values assigned according to Table 2 above described in this document.

As an example, the following data was collected for a male patient with a right knee joint pathology:
Age: 53
In addition, genetic profiling was performed for the polymorphisms rs2665802 of the GH1 gene, rs4880 of the SOD2 gene, rs660339 of the UCP2 gene, rs1260326 of the GCKR gene, rs2229765 of the IGFR-1 gene, rs1870377 of the VEGFR2 gene, rs1042714 of the ADRB2 gene, obtaining the following genetic profile:

| | |
|---|---|
| rs2665802: | A:T |
| rs4880: | G:A |
| rs660339: | C:T |

(continued)

| rs1260326: | C:T |
|---|---|
| rs2229765: | A:G |
| rs1870377: | A:T |
| rs1042714: | C:G |

Once the data were obtained, the β value was obtained for each one, and all the β values were summed to obtain the $B_1x$ value. This value, as well as the assignment of each β value (according to Table 2 previously described in this document) is shown in Table 3.

**Table 3.** β values for the variables collected for the problem patient.

| Variable | Possibility | Beta(β) |
|---|---|---|
| rs2665802 | A:T | 1.606 |
| rs4880 | A:A | 0.107 |
| rs660339 | C:C | 1.415 |
| rs1260326 | C:T | -0.382 |
| rs2229765 | A:A | -1.423 |
| rs1870377 | A:T | -0.110 |
| rs1042714 | C:C | -1.466 |
| Age | 50-69 years | 1.441 |
| $\beta_1X$ =1.187238773 | | |

Once the value $\beta_1x$ = 1.187238773 has been obtained and taking into account that $\beta_0$ = 0.662, the variables with these values are replaced in the formula:

$$P = \frac{1}{1 + e^{-(\beta_o + \beta_1 x)}}$$

obtaining as a result P= 0.86408, i.e. a probability of 86.40% of responding favorably to PRP treatment.

2. Results

Following a methodology as explained above in patients, the algorithm of the invention was applied, i.e.

$$P = \frac{1}{1 + e^{-(\beta_o + \beta_1 x)}}$$

taking into account the different genetic and environmental variables and performing a ROC analysis for each case.

2.1 ROC analysis taking into account the polymorphisms rs2665802 of the GH1 gene, rs4880 of the SOD2 gene, rs660339 of the UCP2 gene, rs1260326 of the GCKR gene, rs2229765 of the IGFR-1 gene, rs1870377 of the VEGFR2 gene and rs1042714 of the ADRB2 gene, and the variable age range.

A ROC analysis was performed (Figure 1), establishing a 69.00% probability of responding to PRP treatment as the cut-off point for classifying the subject or patient in question as a responder or non-responder.

Thus, if the calculated P-value for the subject is greater than 0.69, the subject is classified as highly likely to respond to PRP treatment, while if the P-value is less than 0.69, the subject is classified as likely to respond to PRP treatment.

Taking as an example the male patient for whom a P= 0.86408 was obtained, this value is clearly higher than the selected cut-off point 0.69, therefore, this patient has a high probability of responding to PRP treatment.

The model showed sensitivity values of 81.9% and specificity values of 72.7%. A classification table of patients "Observed" versus "Predicted" by the model (0 being a non-responder and 1 being a responder) is shown below (Table 4).

# EP 4 685 242 A1

**Table 4.** Patient classification table[a]

| Observed | | | Forecast | | |
|---|---|---|---|---|---|
| | | | RESP (0 no and 1 yes) | | Correct percentage |
| | | | 0 | 1 | |
| Step 1 | RESP (0 no and 1 yes) | 0 | 40 | 15 | 72.7 |
| | | 1 | 30 | 136 | 81.9 |
| | Overall percentage | | | | 79.6 |
| a. The cut-off value is .690 | | | | | |

Table 5 gives the value of the area under the curve for the model, with an AUC (area under the curve) of 0.839 and its 95% confidence interval (95% CI) was 0.777-0.902.

**Table 5.** Area under the curve

Test outcome variables: Predicted probability 95% asymptotic confidence

| Area | Error[a] | Asymptotic significance[b] | interv al Lower limit | Upper limit |
|---|---|---|---|---|
| .839 | .032 | .000 | .777 | .902 |

The test outcome variables: Predicted probability have, at a minimum, a tie between the positive true state group and the negative true state group. The statistics could be biased.

a. Under the non-parametric assumption

b. Null Hypothesis: true area = 0.5

2.2 ROC analysis taking into account 2, 3, 4 and 5 genetic variables.

In addition to the ROC analysis taking into account all the genetic variables and the environmental variable 'age' in the previous section, ROC analyses were carried out taking into account the combination of a larger number of variables, demonstrating that they are also useful in the calculation of a subject's probability of responding positively to PRP treatment, as shown in the results obtained and summarised in Table 6, Table 7, Table 8 and Table 9 (located at the end of the Examples).

2.2.1. A genetic variable and the environmental variable age.

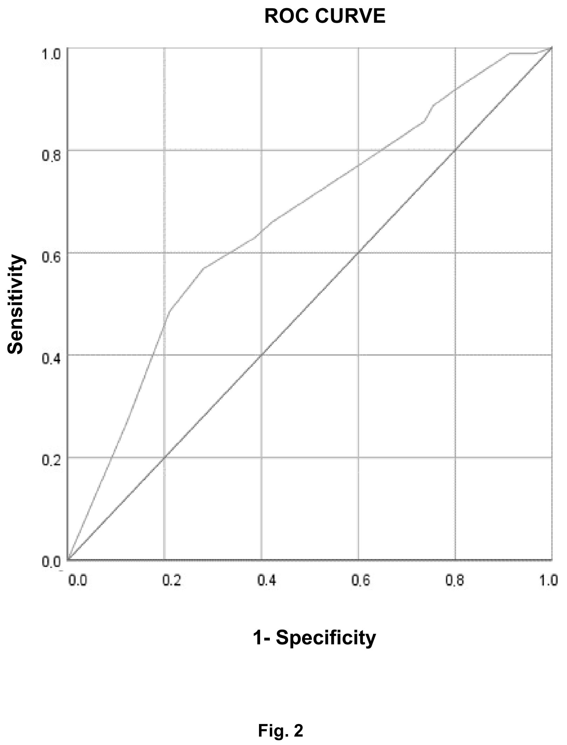

Table 6 shows a summary of the results of the ROC analysis (2 variables) as a function of a genetic variable (polymorphism) and an environmental variable (age) when applying the algorithm. These results are derived from figures 2 to 5:

- Figure 2 shows the ROC curve when applying the algorithm considering as a genetic variable the rs2665802 polymorphism of the GH1 gene and as an environmental variable the age range.

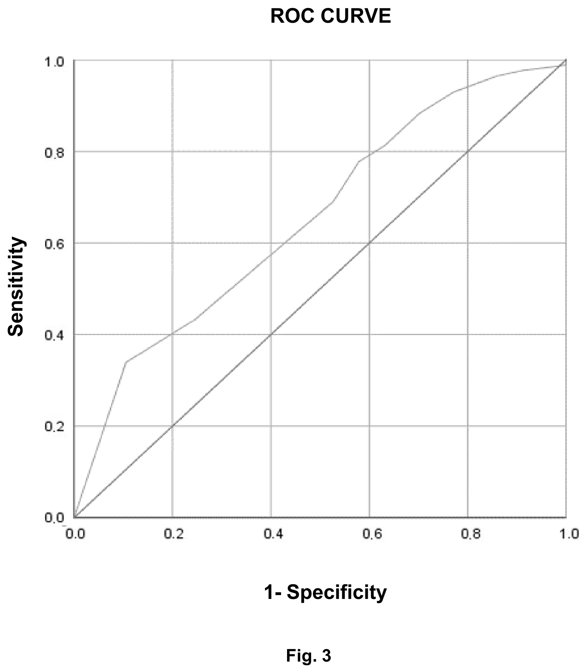

- Figure 3 shows the ROC curve when applying the algorithm considering as a genetic variable the rs4880 polymorphism of the SOD2 gene and as an environmental variable the age range.

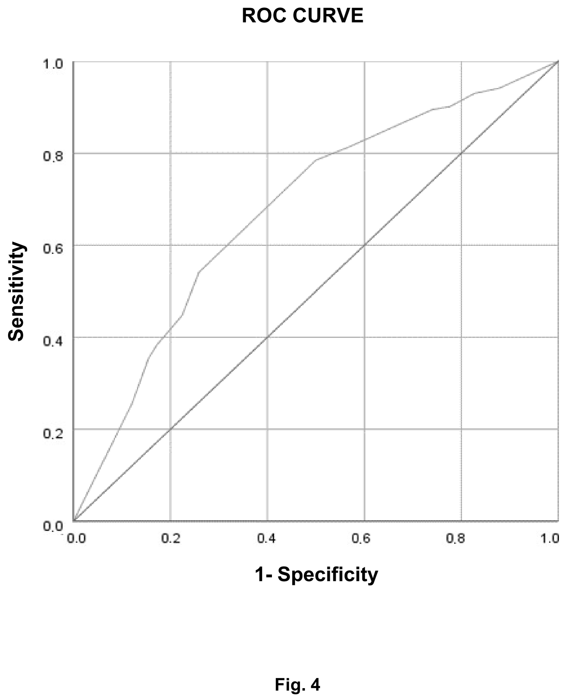

- Figure 4 shows the ROC curve when applying the algorithm considering as a genetic variable the rs660339 polymorphism of the UCP2 gene and as an environmental variable the age range.

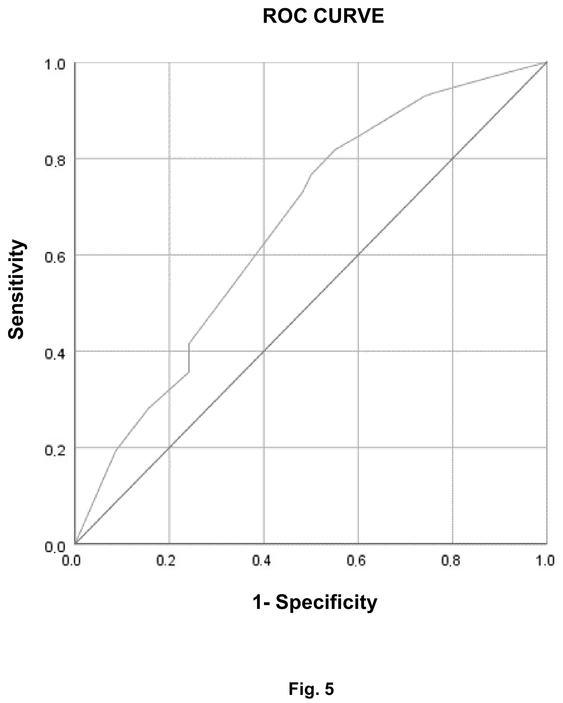

- Figure 5 shows the ROC curve when applying the algorithm considering as a genetic variable the rs1260326 polymorphism of the GCKR gene and as an environmental variable the age range.

2.2.2. Two genetic variables and the environmental variable 'age'.

Table 7 shows a summary of the results of the ROC analysis (3 variables) as a function of two genetic variables (2 polymorphisms) and one environmental variable (age) when applying the algorithm. These results are derived from figures

17

6 to 11:

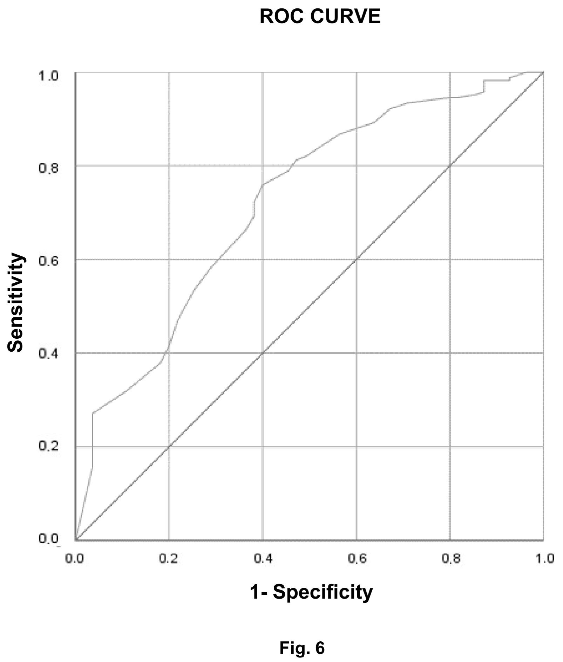

- Figure 6 shows the ROC curve when applying the algorithm considering as genetic variables the genetic polymorphisms rs2665802 of the GH1 gene and rs4880 of the SOD2 gene, and as an environmental variable the age range.

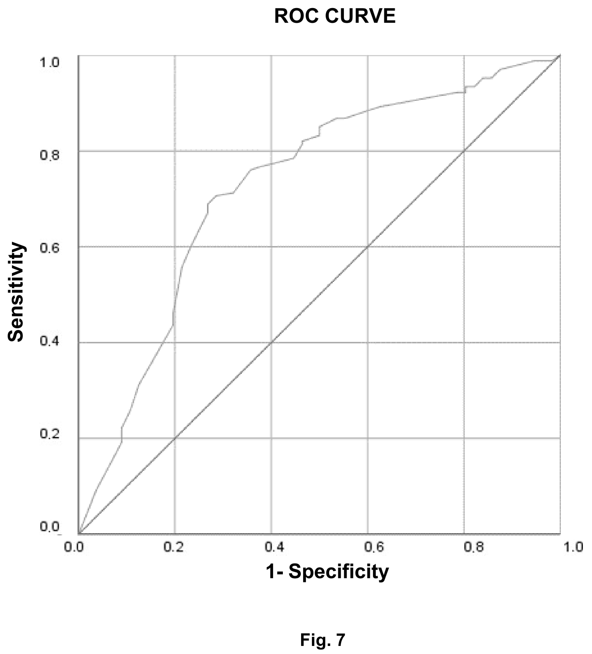

- Figure 7 shows the ROC curve when applying the algorithm considering as genetic variables the genetic polymorphisms rs2665802 of the GH1 gene and rs660339 of the UCP2 gene, and as an environmental variable the age range.

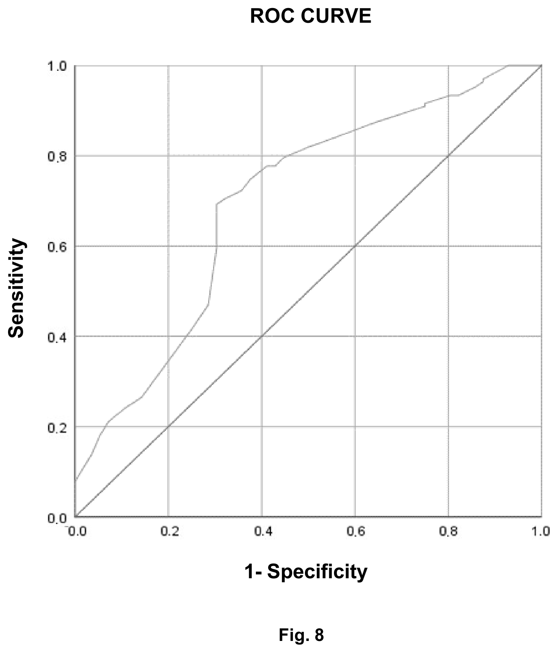

- Figure 8 shows the ROC curve when applying the algorithm considering as genetic variables the genetic polymorphisms rs2665802 of the GH1 gene and rs1260326 of the GCKR gene, and as an environmental variable the age range.

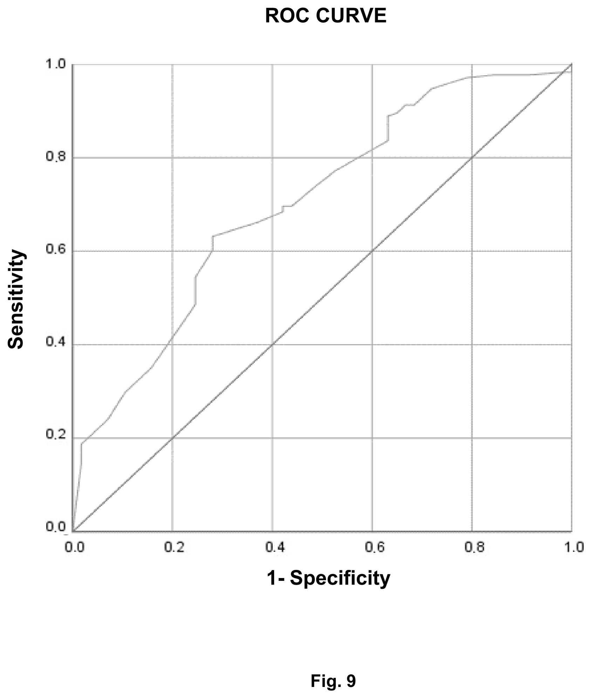

- Figure 9 shows the ROC curve when applying the algorithm considering as genetic variables the genetic polymorphisms rs4880 of the SOD2 gene and rs660339 of the UCP2 gene, and as an environmental variable the age range.

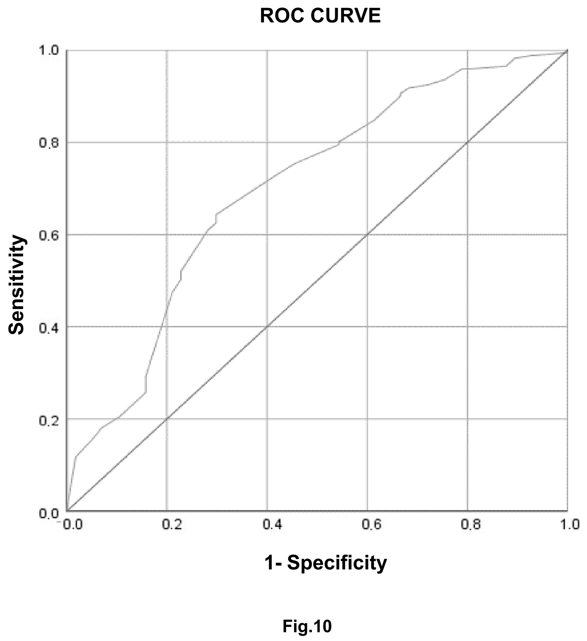

- Figure 10 shows the ROC curve when applying the algorithm considering as genetic variables the genetic polymorphisms rs4880 of the SOD2 gene and rs1260326 of the GCKR gene, and as environmental variable the age range.

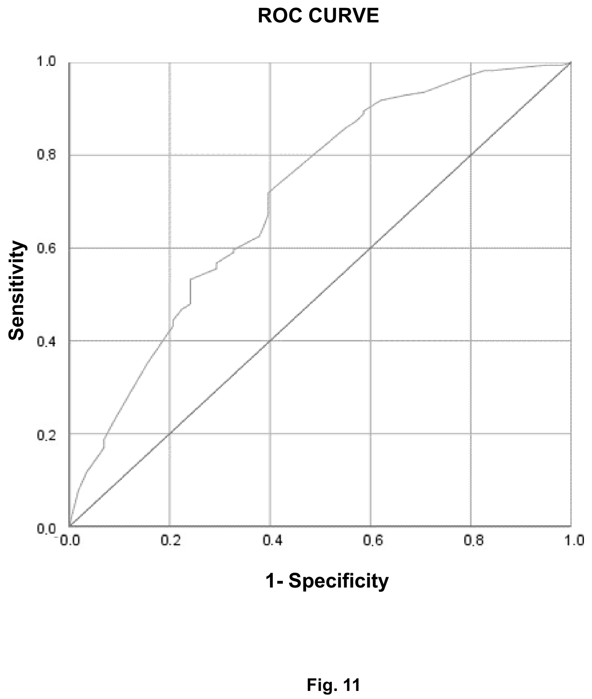

- Figure 11 shows the ROC curve when applying the algorithm considering as genetic variables the genetic polymorphisms rs660339 of the UCP2 gene and rs1260326 of the GCKR gene, and as an environmental variable the age range.

2.2.3. Three genetic variables and the environmental variable 'age'.

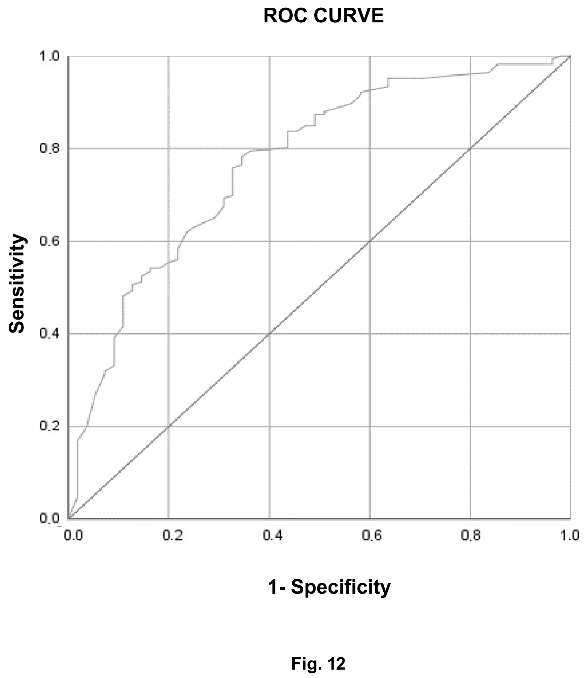

Table 8 shows a summary of the results of the ROC analysis (4 variables) as a function of 3 genetic variables and the environmental variable 'age' considered when applying the algorithm. These results are derived from figures 12 to 15:

- Figure 12 shows the ROC curve when applying the algorithm considering as genetic variables the genetic polymorphisms rs2665802 of the GH1 gene, rs4880 of the SOD2 gene and rs660339 of the UCP2 gene, and as an environmental variable the age range.

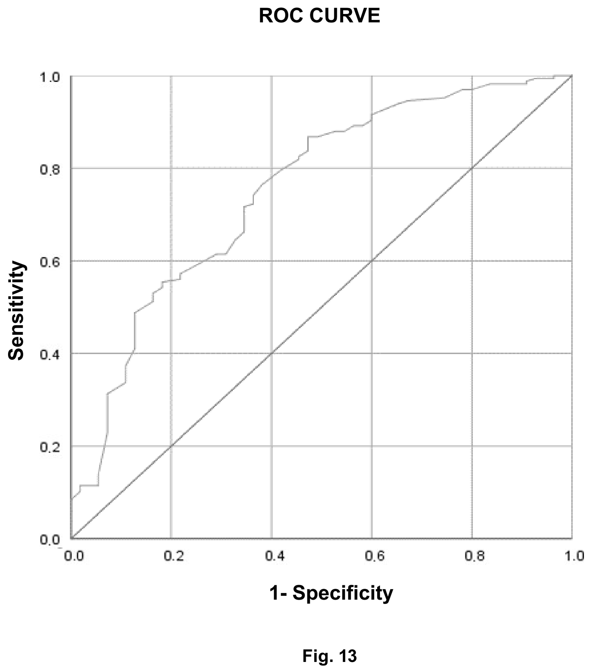

- Figure 13 shows the ROC curve when applying the algorithm considering as genetic variables the genetic polymorphisms rs2665802 of the GH1 gene, rs4880 of the SOD2 gene and rs1260326 of the GCKR gene, and as an environmental variable the age range.

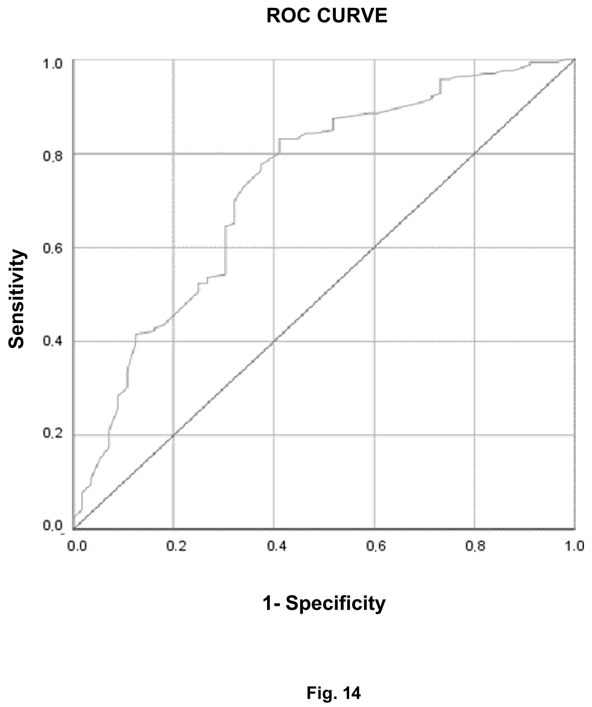

- Figure 14 shows the ROC curve when applying the algorithm considering as genetic variables the genetic polymorphisms rs2665802 of the GH1 gene, rs660339 of the UCP2 gene and rs1260326 of the GCKR gene, and as an environmental variable the age range.

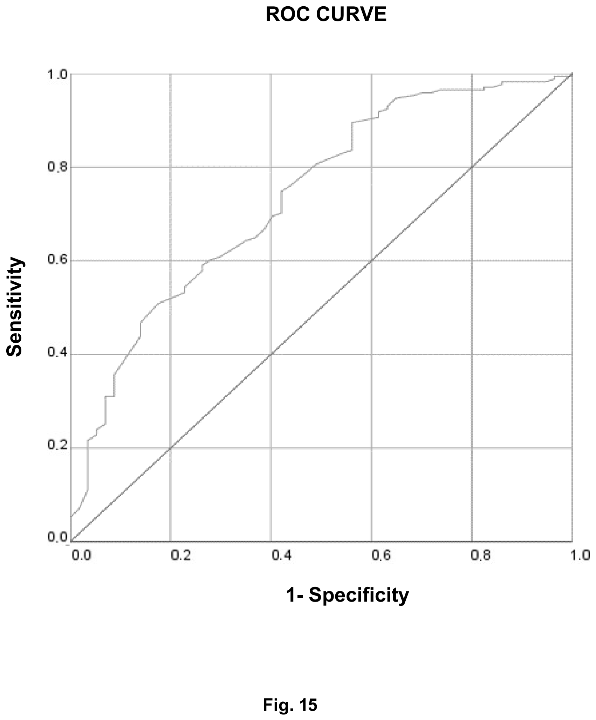

- Figure 15 shows the ROC curve when applying the algorithm considering as genetic variables the genetic polymorphisms rs4880 of the SOD2 gene, rs660339 of the UCP2 gene and rs1260326 of the GCKR gene, and as an environmental variable the age range.

2.2.4. Four genetic variables and the environmental variable 'age'.

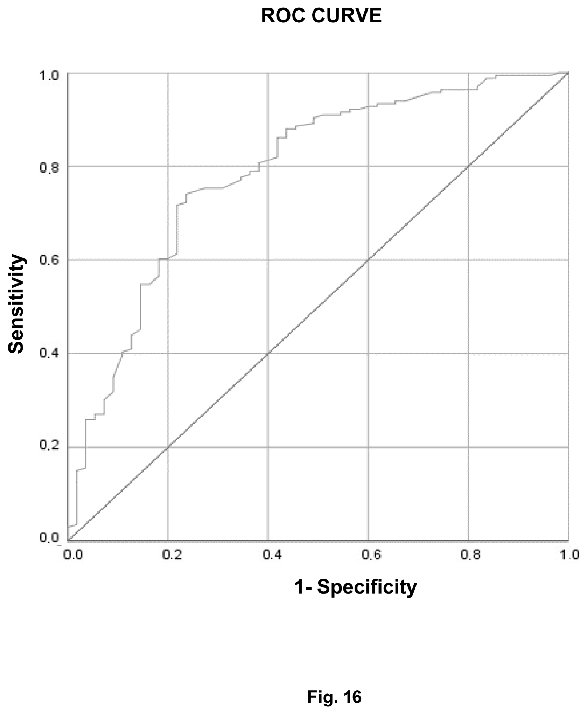

Table 9 (next page) shows a summary of the results of the ROC analysis (5 variables) as a function of 4 genetic variables and the environmental variable 'age' considered when applying the algorithm. These results are derived from Figure 16:

- Figure 16 shows the ROC curve when applying the algorithm considering as genetic variables the genetic polymorphisms rs2665802 of the GH1 gene, rs4880 of the SOD2 gene, rs660339 of the UCP2 gene and rs1260326 of the GCKR gene, and as an environmental variable the age range.

Tables 6, 7, 8 and 9 are shown on the following pages.

**Table 6.** Summary of the results of the (2 variables) as a function of the genetic and environmental variables considered when applying the algorithm.

| | | 1 environmental factor (AGE) vs 1 polymorphism (genetic variable) | | | | | | |
|---|---|---|---|---|---|---|---|---|
| | | GENE | POLYMORPHISM | % Total Prognosticated | Cutoff Value | Specificity | Sensitivity | Curve Area ROC |
| AGE | | GH1 | rs2665802 | 62.5 | 0.718 | 62.9 | 61.4 | 0.662 |
| | | SOD2 | rs4880 | 57.5 | 0.758 | 56.1 | 61.4 | 0.654 |
| | | UCP2 | rs660339 | 71.3 | 0.692 | 78.5 | 50 | 0.677 |
| | | GCKR | rs1260326 | 67.7 | 0.757 | 73.1 | 51.7 | 0.66 |

**Table 7.** Summary of the results of the ROC analysis (3 variables) according to the genetic and environmental variables taken into account when applying the algorithm.

| | | 1 environmental factor (AGE) vs 2 polymorphisms | | | | | | |
|---|---|---|---|---|---|---|---|---|
| | | GENE | POLYMORPHISM | % Total Prognosticated | Cutoff Value | Specificity | Sensitivity | Curve Area ROC |
| AGE | | GH1 | rs2665802 | 69.7 | 0.710 | 72.3 | 61.8 | 0.719 |
| | | SOD2 | rs4880 | | | | | |
| | | GH1 | rs2665802 | 73.1 | 0.710 | 76.6 | 62.5 | 0.726 |
| | | UCP2 | rs660339 | | | | | |
| | | GH1 | rs2665802 | 70.3 | 0.707 | 72.3 | 64.3 | 0.696 |
| | | GCKR | rs1260326 | | | | | |
| | | SOD2 | rs4880 | 65.4 | 0.723 | 63.2 | 71.9 | 0.702 |
| | | UCP2 | rs660339 | | | | | |
| | | SOD2 | rs4880 | 65.8 | 0.729 | 64.3 | 70.2 | 0.699 |
| | | GCKR | rs1260326 | | | | | |
| | | UCP2 | rs660339 | 69.0 | 0.693 | 71.9 | 60.3 | 0.708 |
| | | GCKR | rs1260326 | | | | | |

**Table 8.** Summary of the results of the ROC analysis (4 variables) according to the genetic and environmental variables considered when applying the algorithm.

| | | 1 environmental factor (AGE) vs 3 polymorphisms | | | | | | |
|---|---|---|---|---|---|---|---|---|
| | | GENE | POLYMORPHISM | % Total Prognosticated | Cutoff Value | Specificity | Sensitivity | Curve Area ROC |
| AGE | | GH1 | rs2665802 | 73.8 | 0.72 | 75.9 | 67.3 | 0.77 |
| | | SOD2 | rs4880 | | | | | |
| | | UCP2 | rs660339 | | | | | |
| | | GH1 | rs2665802 | 72.9 | 0.71 | 76.5 | 61.8 | 0.75 |
| | | SOD2 | rs4880 | | | | | |
| | | GCKR | rs1260326 | | | | | |
| | | GH1 | rs2665802 | 71.2 | 0.71 | 72.9 | 66.1 | 0.73 |
| | | UCP2 | rs660339 | | | | | |
| | | GCKR | rs1260326 | | | | | |

(continued)

| | 1 environmental factor (AGE) vs 3 polymorphisms | | | | | | |
|---|---|---|---|---|---|---|---|
| | GENE | POLYMORPHISM | % Total Prognosticated | Cutoff Value | Specificity | Sensitivity | Curve Area ROC |
| | SOD2 | rs4880 | 71.1 | 0.71 | 76.0 | 56.1 | 0.73 |
| | UCP2 | rs660339 | | | | | |
| | GCKR | rs1260326 | | | | | |

**Table 9.** Summary of the results of the ROC analysis (5 variables) according to the genetic and environmental variables considered when applying the algorithm.

| | 1 environmental factor (AGE) vs 4 polymorphisms | | | | | | |
|---|---|---|---|---|---|---|---|
| | GENE | POLYMORPHISM | % Total Prognosticated | Cutoff Value | Specificity | Sensitivity | Curve Area ROC |
| AGE | GH1 | rs2665802 | 73.3 | 0.8 | 72.3 | 76.4 | 0.788 |
| | SOD2 | rs4880 | | | | | |
| | UCP2 | rs660339 | | | | | |
| | GCKR | rs1260326 | | | | | |

**Claims**

1. An *in vitro* method of obtaining data useful for estimating the probability of a subject responding positively to Platelet Rich Plasma (PRP) treatment, comprising the following steps:

   (a) analyze in an isolated biological sample from the subject, at least, one genetic polymorphism selected from the list consisting of rs2665802 of the GH1 gene, rs4880 of the SOD2 gene, rs660339 of the UCP2 gene, and rs1260326 of the GCKR gene, where each such polymorphism may be replaced by another polymorphism that is in linkage disequilibrium with it;
   (b) collect the environmental variable 'age' of the subject,
   (c) assign a $\beta$-value to the genetic polymorphisms analysed in step (a) according to Table 2, and assign a $\beta$-value to the subject's environmental variable collected in step (b) according to Table 2; and
   (d) calculate a subject's probability P of responding positively to PRP treatment, by applying the algorithm:

$$P = \frac{1}{1 + e^{-(\beta_o + \beta_1 x)}}$$

   wherein,

   $\beta_0$ is 0.662, and
   $B_1 x$ is the sum of all $\beta$ values assigned in step (c).

2. The method according to claim 1, wherein step (a) comprises analyzing, at least, two genetic polymorphisms selected from the list consisting of rs2665802 of the GH1 gene, rs4880 of the SOD2 gene, rs660339 of the UCP2 gene, and rs1260326 of the GCKR gene wherein each of said polymorphisms can be replaced by another polymorphism that is in linkage disequilibrium with it.

3. The method according to claim 2, wherein step (a) comprises analyzing at least three genetic polymorphisms selected from the list consisting of rs2665802 of the GH1 gene, rs4880 of the SOD2 gene, rs660339 of the UCP2 gene, and rs1260326 of the GCKR gene, wherein each of said polymorphisms can be replaced by another polymorphism that is in linkage disequilibrium with it.

4. The method according to claim 3, wherein step (a) comprises analyzing at least four genetic polymorphisms selected from the list consisting of rs2665802 of the GH1 gene, rs4880 of the SOD2 gene, rs660339 of the UCP2 gene, and rs1260326 of the GCKR gene, wherein each of said polymorphisms can be replaced by another polymorphism that is in linkage disequilibrium with it.

5. The method according to claim 4, wherein the four genetic polymorphisms are rs2665802 of the GH1 gene, rs4880 of the SOD2 gene, rs660339 of the UCP2 gene and rs1260326 of the GCKR gene.

6. The method according to any one of claims 1 to 5, wherein step (a) further comprises analyzing at least one genetic polymorphism selected from the list consisting of rs2229765 of the IGFR-1 gene, rs1870377 of the VEGFR2 gene, and rs1042714 of the ADRB2 gene, wherein each such polymorphism can be replaced by another polymorphism that is in linkage disequilibrium with it.

7. The method according to any one of claims 1 to 6, wherein step (a) comprises, analyzing the genetic polymorphisms rs2229765 of the IGFR-1 gene, rs1870377 of the VEGFR2 gene, rs1042714 of the ADRB2 gene, rs2665802 of the GH1 gene, rs4880 of the SOD2 gene, rs660339 of the UCP2 gene and rs1260326 of the GCKR gene.

8. The method according to any one of claims 1 to 7, wherein the biological sample is from any tissue susceptible to DNA extraction.

9. The method according to any one of claims 1 to 8, wherein the analysis of the polymorphisms carried out in step (a) comprises the use of probes specific for said polymorphisms.

10. The method according to claim 9, wherein the probes are selected from the list consisting of SEQ ID NO: 1, SEQ ID NO: 2, SEQ ID NO: 3, SEQ ID NO: 4, SEQ ID NO: 7, SEQ ID NO: 8, SEQ ID NO: 11, SEQ ID NO: 12, SEQ ID NO: 15, SEQ ID NO: 16, SEQ ID NO: 19, SEQ ID NO: 20, SEQ ID NO: 23, SEQ ID NO: 24, SEQ ID NO: 27, SEQ ID NO: 28, and any combination thereof.

11. The method according to any one of claims 1 to 10, wherein the analysis of the polymorphisms in step (a) is carried out by PCR and/or sequencing.

12. Kit comprising the means necessary to analyze *in vitro,* in a biological sample isolated from a subject, at least one, two, three, four genetic polymorphisms selected from the list consisting of rs2665802 of the GH1 gene, rs4880 of the SOD2 gene, rs660339 of the UCP2 gene, and rs1260326 of the GCKR gene, wherein each of said polymorphisms can be replaced by another polymorphism that is in linkage disequilibrium with it.

13. Kit according to claim 12, further comprising means for analyzing *in vitro,* in the biological sample isolated from the subject, the genetic polymorphisms rs2229765 of the IGFR-1 gene, rs1870377 of the VEGFR2 gene, and/or rs1042714 of the ADRB2 gene, wherein each of said polymorphisms can be replaced by another polymorphism that is in linkage disequilibrium with it.

14. Kit according to claim 12 or 13, comprising the means necessary to analyze *in vitro,* in a biological sample isolated from the subject, the genetic polymorphisms rs2229765 of the IGFR-1 gene, rs1870377 of the VEGFR2 gene, rs1042714 of the ADRB2 gene, rs2665802 of the GH1 gene, rs4880 of the SOD2 gene, rs660339 of the UCP2 gene and rs1260326 of the GCKR gene.

15. Kit according to any one of claims 12 to 14, wherein the means required to analyze the genetic polymorphisms comprise primers and/or probes.

16. Kit according to claim 15, wherein the primers and/or probes comprise a nucleotide sequence selected from the list consisting of the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 16, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, and/or any combination thereof.

17. Kit according to claim 15, wherein the primers and/or probes comprise a nucleotide sequence selected from the list consisting of the sequences SEQ ID NO: 3, SEQ **ID** NO: 4, SEQ **ID** NO: 7, SEQ ID NO: 8, SEQ ID NO: 11, SEQ ID NO:

12, SEQ **ID** NO: 15, SEQ **ID** NO: 16, SEQ ID NO: 19, SEQ **ID** NO: 20, SEQ ID NO: 23, SEQ ID NO: 24, SEQ ID NO: 27, SEQ ID NO: 28, and any combination thereof.

18. Use of a kit according to any one of claims 12 to 17, in the method according to any one of claims 1 to 11.

## ROC CURVE

Fig. 1

# ROC CURVE

**1- Specificity**

**Fig. 2**

**ROC CURVE**

**1- Specificity**

**Fig. 3**

**ROC CURVE**

Fig. 4

# ROC CURVE

Fig. 5

## ROC CURVE

Fig. 6

## ROC CURVE

Fig. 8

**ROC CURVE**

Fig. 9

ROC CURVE

Fig.10

# ROC CURVE

**Fig. 11**

# ROC CURVE

Fig. 12

ROC CURVE

Fig. 13

## ROC CURVE

Fig. 14

## ROC CURVE

**Fig. 15**

ROC CURVE

Fig. 16

# INTERNATIONAL SEARCH REPORT

| | International application No |
|---|---|
| | PCT/ES2024/070184 |

## A. CLASSIFICATION OF SUBJECT MATTER
INV. C12Q1/6883    G16B20/20
ADD.

According to International Patent Classification (IPC) or to both national classification and IPC

## B. FIELDS SEARCHED

Minimum documentation searched  (classification system followed by classification symbols)

C12Q  G06F  G16B

Documentation searched other than minimum documentation to the extent that such documents are included  in the fields searched

Electronic data base consulted during the  international search (name of data base and,  where practicable, search terms used)

EPO-Internal, BIOSIS, WPI Data

## C. DOCUMENTS CONSIDERED TO BE RELEVANT

| Category* | Citation of document, with indication,  where appropriate, of the relevant passages | Relevant to claim No. |
|---|---|---|
| X | Specification:  "The BioMark HD System sets a new standard for high-throughput real-time pCr, end-point pCr, and digital pCr, with benefits that are impossible to reproduce using many other conventional pCr systems. Integrated Fluidic Circuit (IFC) technology both prepares and performs thousands of reactions in nano", , 1 January 2011 (2011-01-01), XP093188526, Retrieved from the Internet: URL:https://www.ehu.eus/documents/2458249/2712771/BioMark_HD_System_Specification_Sheeteq.pdf | 12-17 |
| A | page 3 | 1-11,18 |

-/--

| X | Further documents are listed in the  continuation of Box C. | | See patent family annex. |
|---|---|---|---|

* Special categories of cited documents :

"A" document defining the general state of the art which is not considered to be of particular relevance

"E" earlier application or patent but published on or after the international filing date

"L" document which may throw doubts on priority  claim(s) or which is cited to establish the publication date of another  citation or other special reason (as specified)

"O" document referring to an oral disclosure, use,  exhibition or other means

"P" document published prior to the international filing date but later than the priority date claimed

"T" later document published after the international filing date or priority date and not in conflict with the application but cited to understand the principle or theory underlying the invention

"X" document of particular relevance;; the claimed invention cannot be considered novel or cannot be considered to involve an inventive step when the document is taken alone

"Y" document of particular relevance;; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 26 July 2024 | 10/09/2024 |

| Name and mailing address of the ISA/ | Authorized officer |
|---|---|
| European Patent Office, P.B. 5818 Patentlaan 2 NL - 2280 HV Rijswijk Tel. (+31-70) 340-2040, Fax: (+31-70) 340-3016 | Tilkorn, A |

Form PCT/ISA/210 (second sheet) (April 2005)

## INTERNATIONAL SEARCH REPORT

| International application No |
|---|
| PCT/ES2024/070184 |

**C(Continuation).    DOCUMENTS CONSIDERED TO BE RELEVANT**

| Category* | Citation of document, with indication, where appropriate, of the relevant passages | Relevant to claim No. |
|---|---|---|
| A | SZYLUK KAROL ET AL: "Polymorphic Variants of the PDGFRB Gene Influence Efficacy of PRP Therapy in Treating Tennis Elbow: A Prospective Cohort Study", JOURNAL OF CLINICAL MEDICINE, vol. 11, no. 21, 28 October 2022 (2022-10-28), page 6362, XP093164527, CH ISSN: 2077-0383, DOI: 10.3390/jcm11216362 abstract | 1-18 |
| A | NACOPOULOS CLEOPATRA ET AL: "Telomere length and genetic variations affecting telomere length as biomarkers for facial regeneration with platelet-rich fibrin based on the low-speed centrifugation concept", JOURNAL OF COSMETIC DERMATOLOGY, vol. 18, no. 1, 15 May 2018 (2018-05-15), pages 408-413, XP093188924, GB ISSN: 1473-2130, DOI: 10.1111/jocd.12666 Retrieved from the Internet: URL:https://onlinelibrary.wiley.com/doi/fu ll-xml/10.1111/jocd.12666> abstract | 1-18 |
| A | Rossano: "Correlation between individual inflammation genetic profile and platelet rich plasma efficacy in hair follicle regeneration: a pilot study reveals prognostic value of IL-1a polymorphism", , 1 November 2017 (2017-11-01), XP093188915, Retrieved from the Internet: URL:https://www.europeanreview.org/wp/wp-c ontent/uploads/5247-5257-Possible-prognost ic-value-of-IL-1alpha-polymorphism.pdf the whole document | 1-18 |

Form PCT/ISA/210 (continuation of second sheet) (April 2005)

## INTERNATIONAL SEARCH REPORT

International application No.

PCT/ES2024/070184

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☒ forming part of the international application as filed.

   b. ☐ furnished subsequent to the international filing date for the purposes of international search (Rule 13ter.1(a)).

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet (1)) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SZYLUK K et al.** *J Clin Med.*, 2022, vol. 11 (21), 6362